(19) Europäisches Patentamt
European Patent Office
Office européen des brevets

(11) **EP 3 336 656 A1**

(12) **EUROPEAN PATENT APPLICATION**

(43) Date of publication:
**20.06.2018 Bulletin 2018/25**

(51) Int Cl.:
***G06F 3/01*** *(2006.01)*     ***A61B 5/16*** *(2006.01)*
***G06Q 50/00*** *(2012.01)*     ***G06Q 99/00*** *(2006.01)*

(21) Application number: **16205170.0**

(22) Date of filing: **19.12.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: OFFIS e.V.
**26121 Oldenburg (DE)**

(72) Inventors:
• **Feuerstack, Sebastian
26122 Oldenburg (DE)**
• **Wortelen, Bertram
26127 Oldenburg (DE)**

(74) Representative: **Schmelcher, Thilo
RCD-Patent
Postfach 3106
52118 Herzogenrath (DE)**

(54) **MODEL BASED DETECTION OF USER REACTION TIMES AND FURTHER EFFECTS AS WELL AS SYSTEMS THEREFORE**

(57) The invention pertains to a Model based detection of attention distribution and/or reaction times and further effects of human-machine interfaces, comprising for a plurality of test-persons the steps of
• Determining and labeling (S100) areas of interest (AOI) and their associated parameters (P), which are selected from the group comprising the expectancy of information appearing within the AOI, the value of all tasks for which the information appearing within the AOI might be relevant, and the relevance of the information appearing within the AOI for each of the aforementioned tasks, per test-person of a human-machine-interface to be evaluated,
• Clustering (S200) the determined areas of interest (AOI) and their associated parameters (P: information expectancy, task value, relevance) for a plurality of test-persons, based on the determined area and/or their labels and/or their associated parameters (P) into classes of area of interest (AOI-classes),
• Simulating (S300) gaze behavior and reaction times per test person based on areas of interest (AOI) and associated parameters (P) defined by the respective person,
• Grouping of simulated eye fixations and saccades for a plurality of test persons according to the determined classes of area of interest (AOI-classes), whereby based on said data for a plurality of test persons probabilistic predictions relating to the distribution of visual attention and/or gaze transitions and/or reaction times are generated,
• Visualizing predictions and determining the causes for predicted distribution of visual attention and reaction times, by analyzing and visualizing associated parameters (P: information, expectancy, task value, relevance) of areas of interest (AOI) for a plurality of test persons,
• Identifying (S600) inconsistent input data, concerning definitions of areas of interest (AOI) and definitions of associated parameters (P) for multiple test persons to identify potentially different understandings of the evaluated system across test persons, whereby identified inconsistencies indicate potentially unexpected behavior of future users of the evaluated system.

Fig. 1

**Description**

BACKGROUND

It is known that man-machine-interfaces need careful design in order to achieve good interaction. That is, even in stress-situation a machine needs to provide necessary information to be interpreted by a user in a manner that said user is able to perceive the information and thereupon may act. This is particular crucial where false handling of a machine or misinterpretation or misconception of information may lead to harmful events.

Such man-machine interfaces may be e.g. a user interface of a machine, a vehicle, a power plant, etc. to mention a view.

As some of this environments may have significant impact, it is often required that these Man-Machine-Interfaces need to be tested and certified.

This is because it is known that risks related to the interaction with a user are mainly influenced by the design of the user-interface.

Therefore, testing of the distribution of (limited) visual attention and determination of average reaction times of a user thereof in respect of unexpected events are performed.

A user of a man-machine-interface may be understood (but not limited to) as a vehicle driver, personal controlling plants, personal controlling grids, control room staff, emergency rooms, operating theater, etc.

Typically testing is performed by usage of (late stage) prototypes of new or alternatives interface designs to determine whether the new or alternative design impacts reaction times towards unexpected events respectively how the gaze is altered with respect to other tasks such as deviating from other tasks such as controlling a vehicle to stay on track.

Situations are in particular critical situations, in which reaction time and or distraction may have severe consequences.

Until today, this was a complex and time consuming process.

In particular two types of process were known to calculate gaze behavior.

> (1) Eye-tracking of a user during interaction with a MMI

> (2) Cognitive modeling by forecasting of gaze behavior on basis of expertise and psychological plausible models.

By means of eye-tracking, gaze behavior of a user is recorded and made available for empirical studies. Eye-tracking allows for a direct measurement of reaction times in response to an unexpected event for the user. Including numerous users, eye-tracking allows for valid determinations of effects of a change in design of the MMI. Validity is thereby closely related to the number of different users.

In such an eye-tracking set-up either a real machine and its interface or a close-to-real simulation is required, which is both time consuming and expensive.

By means of Cognitive modeling prognosis of gaze behavior is based on expertise and findings derived from perception studies with respect to certain parameters of an MMI. Literature mentions perceptual salience, the value of information for the machine controlled by the interface, the physiological effort for perceiving certain information, the expectancy of perceiving new information as parameters of interest. A well known model which is based on these parameters is the SEEV Model described in Christopher D. Wickens et al. "Testing an Attentional Expected Value Model of Visual Scanning", Technical Report ARL-01-14/NASA-01-7, University of Illinois, Aviation Research Lab, Institute of Aviation, November 2001. Alternatives thereto relating to the expectancy of information and the value of information only are known using the Analytic Hierarchy Process as described in Ha, Jun Su, & Seong, Poong Hyun (Oct 2014), "Experimental investigation between attentional-resource effectiveness and perception and diagnosis in nuclear power plants", Nuclear Engineering and Design, 758-772. doi:101016/jnucengdes201408025, lens model as described in "Ecological Validity as a Mediator of Visual Attention Allocation in Human-Machine Systems" by Sarah Miller et al., Technical Report AHFD-04-17/NASA-04-6, University of Illinois, Aviation Research Lab, Institute of Aviation, December 2004 and the model described by Carbonell in "A Queueing Model of Many-Instrument Visual Sampling", Published in: IEEE Transactions on Human Factors in Electronics, Volume: HFE-7, Issue: 4, Dec. 1966, Page(s): 157 - 164, DOI: 10.1109/THFE.1966.23298.

Since no standard procedure for determination of the parameter is established, quality of the forecast is particular subject to the expertise of the modeling person.

The approaches known so far encompass several drawbacks.

For example, eye-tracking methods require a quite realistic set-up in order to achieve meaningful results. This however is time-consuming and extremely expensive (e.g. suppose interfaces of a power plant control room, vehicle simulators, etc.).Most often such a interface testing may only be performed in a very late design stage, e.g. relaying on prototypes and real interaction. Hence, even though the testing is useful, it may be that the overall design may no longer be changed such that the results achieved may only lead to partial improvement within the constraints of a late development stage. Furthermore, data acquisition is typically time-consuming as due o the high costs typically only a limited number of set-ups, sometimes only one, may be available which therefore affords a sequential operation in view of a plurality of users. In addition, eye-tracking does not allow to gather data from all users, but requires certain physical conditions such that visual impairments (glasses, Ptosis, etc.) may lead to exclusion of a user for eye-tracking. It is also known that in highly dynamic systems, e.g. vehicle simulators, data analysis is extremely complex as the data acquired needs to be analyzed along data relating to moving objects necessitating a highly synchronized data scheme. Even then a major drawback of eye-tracking-systems is that from a conceptual approach they do not allow for analyzing reasons for a certain gaze pattern but only for tracking a certain effect.

But also the cognitive modeling shows certain draw-backs as they require a very specific expertise to achieve valid forecasts, as these approaches are very sensitive to small conceptual errors. In addition these models tend to provide a very poor reproducibility. As these models a user centric, they do not allow for scalability. I.e. models provided by different modeling persons may not be combined. It is noted that because of the possibility to aggregate eye-tracking data of a plurality of users, eye-tracking-systems do not suffer from a lack of scalability.

Based on the above findings, the invention aims at solving at least some of the problems.

BRIEF DESCRIPTION OF THE INVENTION

The invention therefore proposes a model based detection of attention distribution and/or reaction times and further effects of human-machine interfaces, comprising for a plurality of test-persons the step of determining and labeling areas of interest and their associated parameters, which are selected form the group comprising expectancy of information appearing within the areas of interest, value of all tasks for which the information appearing within the areas of interest might be relevant, and relevance of the information appearing within the areas of interest for each of the aforementioned tasks, per test-person of a human-machine-interface to be evaluated, the step of clustering the determined areas of interest and their associated parameters such as information expectancy, task value, relevance for a plurality of test-persons, based on the determined area and/or their labels and/or their associated parameters into classes of area of interest, the step of simulating gaze behavior and reaction times per test person based on areas of interest and associated parameters defined by the respective person, the step of grouping of simulated eye fixations and saccades for a plurality of test persons according to the determined classes of area of interest, whereby based on said data for a plurality of test persons probabilistic predictions relating to the distribution of visual attention and/or gaze transitions and/or reaction times are generated, the step of determining the causes for predicted distribution of visual attention and reaction times, by analyzing and visualizing associated parameters of areas of interest for a plurality of test persons, and the step of identifying inconsistent input data, concerning definitions of areas of interest and definitions of associated parameters for multiple test persons to identify potentially different understandings of the evaluated system across test persons, whereby identified inconsistencies indicate potentially unexpected behavior of future users of the evaluated system.

The invention also proposes a system for performing model based detection of attention distribution and/or reaction times and further effects

BRIEF DESCRIPTION OF THE FIGURE

Fig. 1 shows a high-level comparison of methodical steps of an eye-tracking-system according to the state of the art versus methodical steps within a system and method according to embodiments of the invention,

Fig. 2 shows more details of methodical steps within a system and method according to embodiments of the invention, and

Fig. 3 a schematic representation of a system according to the invention.

DETAILED DESCRIPTION

The present disclosure describes preferred embodiments with reference to the Figures, in which like reference signs represent the same or similar elements. Reference throughout this specification to "one embodiment," "an embodiment," or similar language means that a particular feature, structure, or characteristic described in connection with the embodiment is included in at least one embodiment of the present invention. Thus, appearances of the phrases "in one embodiment," "in an embodiment," and similar language throughout this specification may, but do not necessarily, all refer to the same embodiment.

The described features, structures, or characteristics of the invention may be combined in any suitable manner in one or more embodiments. In the description, numerous specific details are recited to provide a thorough understanding of embodiments of the invention.

I.e., unless indicated as alternative only any feature of an embodiment may also be utilized in another embodiment.

In addition, even though at some occurrences certain features will be described with reference to a single entity, such a description is for illustrative purpose only and actual implantations of the invention may also comprise one or more of these entities. I.e. usage of singular also encompasses plural entities unless indicated.

Within the model based method of detection of attention distribution and/or reaction times and further effects of human-machine interfaces the approach is different than to the known eye-tracking-systems.

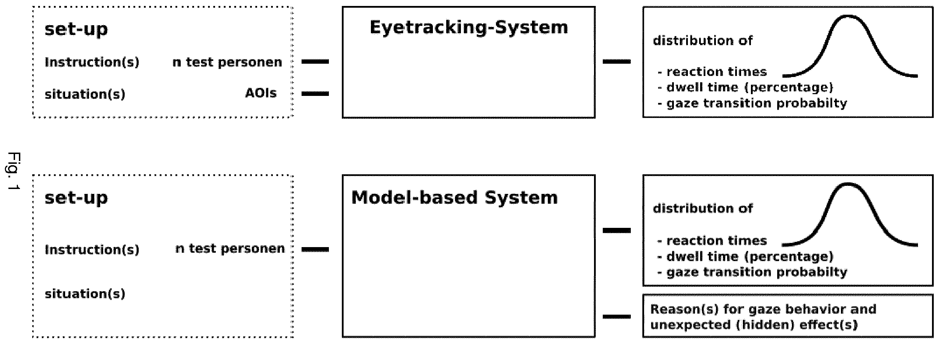

To illustrate this, we refer to Figure 1 showing a high level schematic comparison. In the upper half of the Figure a set-up of a conventional eye-tracking scheme is shown. As detailed above a complex hardware set-up needs to be provided to realistically reproduce a certain situation. For each situation a set of instructions needs to be defined. Beforehand, that is irrespective of the test person, a set of areas of interest is defined. Then, within the given set-up a number n of test persons are subjected to the situation. During this test, the test persons are closely monitored by the eye-tracking-system with respect to the situation and the pre-determined Areas of Interest. Once, the test is completed for a number n of test persons, a distribution of reaction times and/or dwell time percentage and/or gaze transition probabilities may be determined. As can be understood by means of the pre-determined AOIs, the methodical approach can only provide results to what is defined by the modelling person.

In contrast, the approach of the invention is completely different. There, no eye-tracking is necessary. Rather, a situation may be visualized on a computer screen or by means of projecting. However, the model based approach differs in further aspects, namely that there are no pre-defined AOIs. Therefore, the system and methods according to the invention does not only allow to provide for a number n of test persons, a distribution of reaction times and/or dwell time percentage and/or gaze transition probabilities as in known systems but also allows to determine reasons for a particular gaze behavior as well as for determining unexpected (hidden) effects.

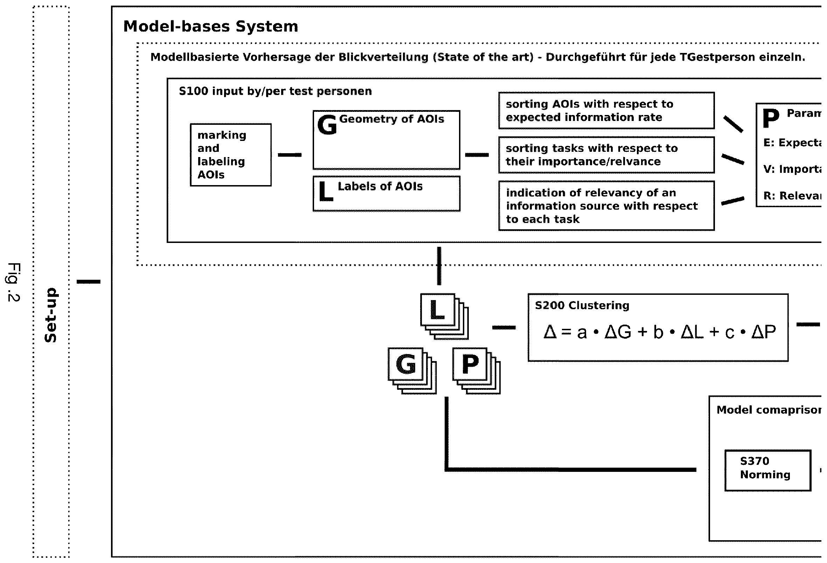

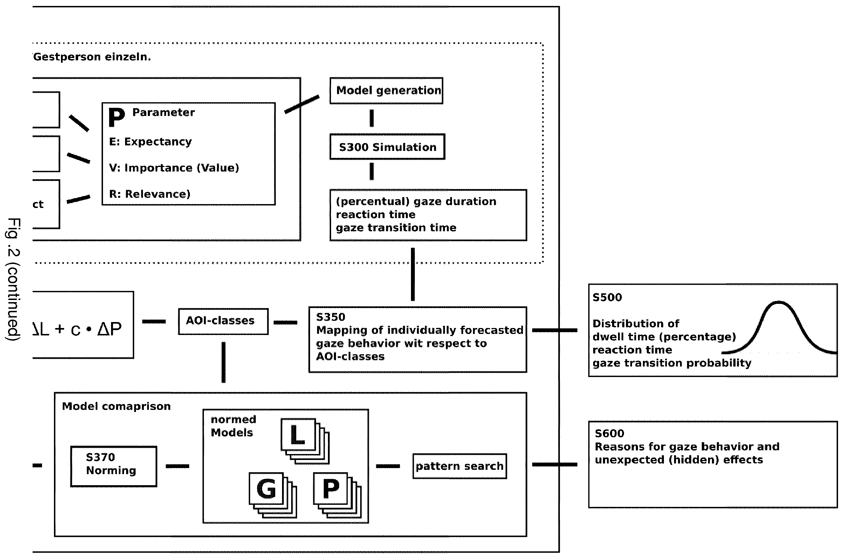

Let's have a closer look. Fig. 2 is detailing the inventive approach. According to the invention for a plurality of test-persons certain steps are performed.

As a first step within a given situation and having a certain instruction at hand, the test person each determines and labels S100 areas of interest based on the situation and the instruction(s). The result of this marking and labeling is a set of Geometry G of AOIs with respect to said given situation and the respective labels L. For each of said AOIs further parameter is/are associated. These parameter P may be selected from a group comprising an expectancy of information appearing within a particular AOI, a value of all tasks for which the information appearing within a particular AOI might be relevant, and a relevance of the information appearing within a particular AOI for each of the aforementioned tasks.

This determination of AOIs and their associated parameters is performed for each test-person of a human-machine-interface to be evaluated.

A set of geometry G as indicated above may be any kind of suitable parameters indicating an area of interest. E.g. it may be coordinates and/or alignment data of a rectangle or an ellipse or any other shape useful for uniquely identifying an area of interest, e.g. on a display DIS. A label L may be associated to each AOI allowing for easy re-usage of an area of interest within different situations without the necessity to (re-) assign an AOI.

Hence, as the test persons are individuals, it is to be expected that each test person determines at least slightly deviating sets of AOIs and associated parameter. Also, due to the effect that no longer a modelling person is defining the AOIs also inexperienced users having no or only limited knowledge of cognitive modelling are able to define AOIs. That is instead of the knowledge of the modelling person - so called human factors experts- , the deep knowledge of test persons actually interacting with machines, i.e..the so called domain experts, is made available.

Thereby the invention is no longer dependent on the expertise of the modelling person predefining a set of AOIs but each test person is determining an own model.

Once for a certain number n of test persons said AOIs and associated parameters are determined, in a next step the determined areas of interest AOI and their associated parameters P, e.g. information expectancy, task value, relevance, for a plurality of test-persons are clustered S200, based on the determined area G and/or their labels L and/or their associated parameters P into classes of area of interest AOI-classes.

The clustering may be a learning process. It allows for aggregation, norming and comparing models developed by each test person. By means of e.g. a pattern search in a step 470 hidden effects may be detected.

Clustering involves detecting AOI classes. As the test persons are different, the set of AOIs per test person is different. However, it is likely that certain information sources are determined by plural test persons even though the labels L used and/or the geometry G and/or associated parameter P might be (slightly) different. By means of a cluster analysis, e.g. a hierarchical clustering necessitating a distance function, AOI classes are determined. E.g. in case of hierarchical clustering a numerical distance of two objects, e.g. two AOIs is determined. This numerical distance may be based on one or more selected from the geometry G, the Label L and the associated parameter P. Obviously when basing the numerical distance on more than one input variable, weighing factors may be employed. A possible function to determine a numerical distance may be as follows:

$$\Delta = a \cdot \Delta G + b \cdot \Delta L + c \cdot \Delta P$$

Here, a, b and c are weighing factors. $\Delta G$ denotes a geometric distance, $\Delta L$ may be semantic relationship of labels L, e.g. Resnik's semantic similarity (see e.g. P. Resnik (1999) "Semantic Similarity in a Taxonomy: An Information-Based Measure and its Application to Problems of Ambiguity in Natural Language", Volume 11, pages 95-130, Journal of Artificial Intelligence Research), and $\Delta P$ denotes a numerical distance in parameter(s) P. As the values associated to AOI parameters may differ in range for each test person, it may be necessary to norm these parameters beforehand in a step 370.

Norming may involve different approaches for different data. In case a test person has not entered an AOI for a particular AOI class, this can be understood as the test person being of the opinion that the AOI class is irrelevant. Hence, one may simply enter default values indicating that the AOI class is irrelevant. In case a test person has associated more than one AOI to an AOI class, i.e. a test person is more specific in detailing, than it may be necessary to aggregate these AOIs into a same AOI class such that e.g. all individual associated parameters P and geometry G are joined under a common label L. Within the joint parameters, the higher ranking ones prevail while the lower ranking ones are dismissed. That is, a parameter of a constituting AOI having a higher relevancy, expectancy, etc. is determining the joint parameter as it would be contradictive if a lower value would be associated than in a constituting AOI.

As some errors might lead to false results, it may be necessary to employ spell checkers in order not to generate large semantic distances $\Delta L$ due to typos. Other options might be that in case of a close match of Geometry G between test persons, the differing labeling L may not be taken into account or to a less significant value (e.g. be adjusting weighing factors) such that typos are less important.

It may also be that geometries G of a same label L, that is $\Delta L=0$, associated by different test persons overlap either completely or in part, that is $\Delta G$ is small. That might be seen as an indication that a certain AOI are member of an AOI class. However, in case the geometries G do not overlap or even are arranged at larger distance, one may deduce that certain information is displayed redundantly. Therefore it is wise to define the weighting factor for $\Delta L$ based on the actual value of $\Delta G$.

By usage of learning algorithms, which learn the weighting factors based on the values of the distance metrics $\Delta G$, $\Delta L$ and $\Delta P$, one may compensate these effects.

Thereafter, in parallel, beforehand, a gaze behavior and reaction times per test person may be simulated S300 based on areas of interest AOI and associated parameters P defined by the respective test person.

After clustering S200 and simulation for a plurality of test persons in S300, it is possible to group simulated eye fixations and saccades for a plurality of test persons according to the determined classes of area of interest AOI-classes, whereby based on said data for a plurality of test persons probabilistic predictions relating to the distribution of visual attention and/or gaze transitions and/or reaction times are generated. It may be beneficial to also perform a mapping in a step 350. That is gaze transitions calculated individually are mapped to the AOI-classes. Gaze data generated by the models of the individual test persons is thereby referenced towards the AOI-classes generated by clustering rather than the individually generated AOI model. Thereby it is provided for a grouping of gaze data per AOI-class. Thereby allowing for providing results known from conventional eye-tracking systems.

Thereafter, in parallel, beforehand, the causes for predicted distribution of visual attention and reaction times may be determined S600 by analyzing and visualizing associated parameters P, such as information, expectancy, task value, relevance of areas of interest AOI for a plurality of test persons as well as inconsistent input data may be identified, concerning definitions of areas of interest AOI and definitions of associated parameters P for multiple test persons to identify potentially different understandings of the evaluated system across test persons, whereby identified inconsistencies indicate potentially unexpected behavior of future users of the evaluated system.

As indicated, models may be compared. That is, due to automatic clustering certain AOIs of individual test persons may be compared to thereby determine a model valid for a maximum of test persons and to detect certain patterns.

Interesting patterns are for example:

- Missing AOIs. Some models may contain AOIs from a certain AOI class that other models lack. This may be a hint towards several issues, e.g.:

    o If the models were created by potential end-users, who were not able to identify all AOIs, then this might point to a non-intuitive design.
    o If a set of models was created by end-users and another set by the MMI designers, then a lack of a certain AOI class in the designers' models may hint towards an information source that the end-users are using, but the designers are not of aware of the end-users using it. Vice versa, a lack of a certain AOI class in the end-users' models may hint at an information source, that the designers think is useful, but actually is not used by the end-users.

- deviating parameter tendencies. This may happen in case different situations are analyzed. Than test persons may have a different understanding of certain information, respective AOI within these different situations. In particular in case of testing different MMIs, this may hint towards a lack of information which information rate and/or relevancy certain information provides within different situations.

That is, due to the pattern search, one may detect certain drawbacks of an MMI, e.g. introduced due to a lack of understanding by the designer which may be a reason for a certain gaze behavior. Due to the nature of the process one is also able to detect a certain parameter as being a source of misunderstanding. E.g. in case of several MMI designs tested by a number of test persons and showing consistent deviations of a certain parameter P, this may be seen as a confirmation that this parameter is a reason for differing gaze behavior. The automatically identified patterns can afterwards be visualized and analyzed S600.

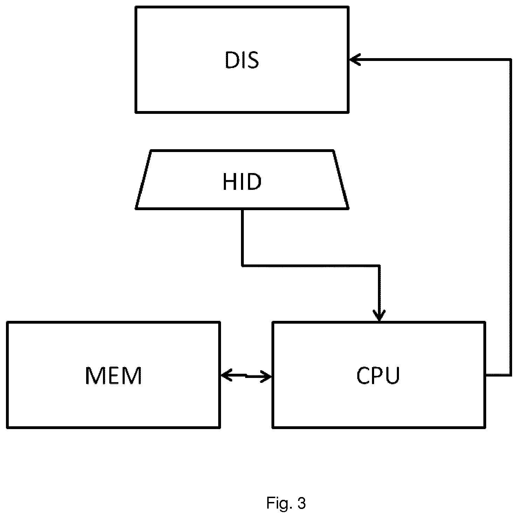

The invention also pertains to a system for performing model based detection of attention distribution and/or reaction times and further effects. Such a system is shown schematically in Fig. 3. The system comprises a visual representation DIS of a human-machine-interface to be evaluated. This visual representation may be a display or a projection such as a computer display or a beamer.

The system also comprises an input-unit HID for receiving input of a test-person regarding areas of interest AOI and their associated Parameters P, i.e. expectancy, task value, relevance. The input-unit may be embodied in a conventional human input device such as a keyboard, a mouse, a trackball, a joystick or the like.

In addition the system comprises a storing unit MEM for storing input of a test-person regarding areas of interest AOI and their associated parameters P of a plurality of test-persons, as well as a processing unit CPU for clustering the determined areas of interest AOI and their expected relevancy P for a plurality of test-persons, based on the determined area and/or the AOI labels and/or their associated parameters into classes of area of interest AOI-classes. The storing unit MEM may be any kind of electronic, magnetic or other storage media such as flash memory, a hard drive or RAM, The processing unit CPU may be any kind of processor, such as microprocessor, an ASIC or an FPGA.

The processing unit CPU subsequently assigns fixations and saccades of simulated gaze behavior to areas of interest AOI per test person according to the determined classes of area of interest AOI-classes, whereby based on said data for a plurality of test persons probabilistic predictions relating to visual attention and/or gaze transitions and/or reaction times are generated, and the processing unit is further adapted to determine the causes for predicted distribution of visual attention and reaction times, by analyzing and visualizing associated parameters for classes of areas of interest AOI-classes for a plurality of test persons, and the processing unit is further adapted to identify inconsistent input data for classes of areas of interest AOI-classes of multiple test persons, concerning their definitions of areas of interest AOI and definitions of associated parameters P to identify potentially different understandings of the evaluated system across test persons.

Obviously, the processing unit CPU may also be arranged to control the visual representation DIS.

As can be seen, the invention allows not only to determine the parameters as known at considerably lower cost and shorter time (since testing of a plurality of test persons may be done in parallel as the set-up is rather inexpensive), the invention also allows to detect other issues.

In particular, since the invention is based on the individual definition of AOIs, it is possible to overcome the drawback of being based on the expertise of the modelling person. In the invention, an AOI of a test person deviating by a certain degree from the respective AOI class may provide an insight that certain information is not available to all test person or that a certain kind of information is held more relevant than another. This may indicate a lack of knowledge or that a certain kind of information may be valuable but was overseen by others. I.e., based on this knowledge one may arrive at the conclusion that an individual person needs to be trained or that a certain kind of information is not made available in a way that it was recognized readily. Hence, this may be understood as indication that a certain kind of information needs to be presented in another (e.g. more prominent) way.

Hence, unlike providing questionnaires afterwards within eye-tracking-systems leading to a blurred subjective interpretation, the system inherently allows to locate such effects in an objective and systematic manner.

That is the invention allows for detecting reasons of a certain gaze behavior. Since the display DIS showing a situation may be arranged at any (remote) place, it is no longer necessary to arrange for on-site measurements as necessary with expensive eye-tracking-systems. Furthermore, test persons so far excluded within eye-tracking can also be included.

It is to be noted that the invention has been described to visual stimuli. It is however understood that the idea of the invention is not limited thereto but may also be used in the context of audio information and audiovisual information.

**Claims**

1.  Model based detection of attention distribution and/or reaction times and further effects of human-machine interfaces, comprising for a plurality of test-persons the steps of

    • Determining and labeling (S100) areas of interest (AOI) and their associated parameters (P), which are selected from the group comprising:

        • the expectancy of information appearing within the AOI,
        • the value of all tasks for which the information appearing within the AOI might be relevant, and
        • the relevance of the information appearing within the AOI for each of the aforementioned tasks,

    per test-person of a human-machine-interface to be evaluated,

• Clustering (S200) the determined areas of interest (AOI) and their associated parameters (P) for a plurality of test-persons, based on the determined area and/or their labels and/or their associated parameters (P) into classes of area of interest (AOI-classes),

• Simulating (S300) gaze behavior and reaction times per test person based on areas of interest (AOI) and associated parameters (P) defined by the respective person,

• Grouping of simulated eye fixations and saccades for a plurality of test persons according to the determined classes of area of interest (AOI-classes), whereby based on said data for a plurality of test persons probabilistic predictions relating to the distribution of visual attention and/or gaze transitions and/or reaction times are generated,

• Visualizing the predicted distribution of visual attention and determining the causes for predicted distribution of visual attention and reaction times, by analyzing and visualizing associated parameters (P) of areas of interest (AOI) for a plurality of test persons,

• Identifying (S600) inconsistent input data, concerning definitions of areas of interest (AOI) and definitions of associated parameters (P) for multiple test persons to identify potentially different understandings of the evaluated system across test persons, whereby identified inconsistencies indicate potentially unexpected behavior of future users of the evaluated system.

2. System for performing model based detection of attention distribution and/or reaction times and further effects, the system comprising a visual representation (DIS) of a human-machine-interface to be evaluated, an input-unit (HID) for receiving input of a test-person regarding areas of interest (AOI) and their associated Parameters (P), a storing unit (MEM) for storing input of a test-person regarding areas of interest (AOI) and their associated parameters (P) of a plurality of test-persons, a processing unit (CPU) for clustering the determined areas of interest (AOI) and their expected relevancy (P) for a plurality of test-persons, based on the determined area and/or the AOI labels and/or their associated parameters into classes of area of interest (AOI-classes), wherein the processing unit (CPU) subsequently assigns fixations and saccades of simulated gaze behavior to areas of interest (AOI) per person according to the determined classes of area of interest (AOI-classes), whereby based on said data for a plurality of test persons probabilistic predictions relating to visual attention and/or gaze transitions and/or reaction times are generated, and the processing unit (CPU) is further adapted to determine the causes for predicted distribution of visual attention and reaction times, by analyzing and visualizing associated parameters (P) for classes of areas of interest (AOI classes) for a plurality of test persons, and the processing unit is further adapted to identify inconsistent input data for classes of areas of interest (AOI classes) of multiple test persons, concerning their definitions of areas of interest (AOI) and definitions of associated parameters (P) to identify potentially different understandings of the evaluated system across test persons.

Fig. 1

**Model-bases System**

Modellbasierte Vorhersage der Blickverteilung (State of the art) - Durchgeführt für jede TGestperson einzeln.

**S100 input by/per test personen**

marking and labeling AOIs

**G** Geometry of AOIs

**L** Labels of AOIs

sorting AOIs with respect to expected information rate

sorting tasks with respect to their importance/relvance

indication of relevancy of an information source with respect to each task

**P** Param

E: Expecta

V: Importa

R: Releva

**L**

**G** **P**

**S200 Clustering**

$$\Delta = a \cdot \Delta G + b \cdot \Delta L + c \cdot \Delta P$$

Model comaprisor

**S370 Norming**

Set-up

Fig .2

EP 3 336 656 A1

9

Gestperson einzeln.

**Model generation**

**S300 Simulation**

(percentual) gaze duration
reaction time
gaze transition time

**P** Parameter

E: Expectancy

V: Importance (Value)

R: Relevance)

$L + c \cdot \Delta P$

ct

**S350**
Mapping of individually forecasted
gaze behavior wit respect to
AOI-classes

AOI-classes

**Model comaprison**

**S370
Norming**

normed
Models

L

G  P

pattern search

**S500**

**Distribution of**
dwell time (percentage)
reaction time
gaze transition probability

**S600**
**Reasons for gaze behavior and**
**unexpected (hidden) effects**

Fig .2 (continued)

Fig. 3

Europäisches
Patentamt

European
Patent Office

Office européen
des brevets

# EUROPEAN SEARCH REPORT

Application Number

EP 16 20 5170

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PINGMEI XU ET AL: "Spatio-Temporal Modeling and Prediction of Visual Attention in Graphical User Interfaces", HUMAN FACTORS IN COMPUTING SYSTEMS, ACM, 2 PENN PLAZA, SUITE 701 NEW YORK NY 10121-0701 USA, 7 May 2016 (2016-05-07), pages 3299-3310, XP058257561, DOI: 10.1145/2858036.2858479 ISBN: 978-1-4503-3362-7 * abstract * * "Procedure"; page 3308 * | 1,2 | INV. G06F3/01 A61B5/16 G06Q50/00 G06Q99/00 |
| X | US 2008/309874 A1 (ZUCCOLOTTO ANTHONY [US] ET AL) 18 December 2008 (2008-12-18) * paragraphs [0047] - [0049]; claims 1,2 * | 1,2 | |
| | | | TECHNICAL FIELDS SEARCHED (IPC) |
| | | | G06F A61B G06Q |

The present search report has been drawn up for all claims

| Place of search | Date of completion of the search | Examiner |
|---|---|---|
| Munich | 16 May 2017 | del Rey Zapatero, M |

EPO FORM 1503 03.82 (P04C01)

## ANNEX TO THE EUROPEAN SEARCH REPORT
## ON EUROPEAN PATENT APPLICATION NO.

EP 16 20 5170

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2017

| Patent document cited in search report | Publication date | Patent family member(s) | Publication date |
|---|---|---|---|
| US 2008309874 A1 | 18-12-2008 | US 2008309874 A1<br>US 2011063570 A1 | 18-12-2008<br>17-03-2011 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHRISTOPHER D. WICKENS et al.** Testing an Attentional Expected Value Model of Visual Scanning. *Technical Report ARL-01-14/NASA-01-7, University of Illinois, Aviation Research Lab, Institute of Aviation,* November 2001
- **HA, JUN SU ; SEONG, POONG HYUN.** Experimental investigation between attentional-resource effectiveness and perception and diagnosis in nuclear power plants. *Nuclear Engineering and Design,* October 2014, 758-772

- **SARAH MILLER et al.** Ecological Validity as a Mediator of Visual Attention Allocation in Human-Machine Systems. *Technical Report AH-FD-04-17/NASA-04-6, University of Illinois, Aviation Research Lab, Institute of Aviation,* December 2004
- A Queueing Model of Many-Instrument Visual Sampling. **CARBONELL.** IEEE Transactions on Human Factors in Electronics. December 1966, vol. HFE-7, 157-164
- **P. RESNIK.** Semantic Similarity in a Taxonomy: An Information-Based Measure and its Application to Problems of Ambiguity in Natural Language. *Journal of Artificial Intelligence Research,* 1999, vol. 11, 95-130